# FASCICULE DE BREVET EUROPEEN

(11) **EP 3 270 886 B1**
(45) Date de publication et mention de la délivrance du brevet: **21.10.2020**
(21) Numéro de dépôt: 16713344.6
(22) Date de dépôt: 18.03.2016
(51) Int. Cl.: A61K 8/9711, A61K 8/97, A61K 36/00, A23L 33/105, A61K 8/9717, A61K 8/9722, A61K 8/9728, A61K 8/9733, A61K 8/9789, A61K 8/9794, A61P 17/00, A61Q 19/00

(54) **PROCEDE DE PREPARATION D'UN EXTRAIT DE MATRICE VEGETALE AVEC UN COMPOSE AMPHIPHILE NON IONIQUE COMME ADJUVANT D'EXTRACTION EN MILIEU AQUEUX**
VERFAHREN ZUR HERSTELLUNG EINES EXTRAKTES EINER MATRIX VON PFLANZLICHEM URSPRUNGS MIT NICHTIONISCHER AMPHIPHILER VERBINDUNG ALS EXTRAKTIONSADJUVANS IN EINEM WÄSSRIGEN MEDIUM
METHOD FOR PRODUCING AN EXTRACT OF A MATRIX OF VEGETABLE ORIGIN WITH A NON-IONIC AMPHIPHILIC COMPOUND AS EXTRACTION ADJUVANT IN AN AQUEOUS MEDIUM

(30) Priorité: 18.03.2015 FR 1552248
(43) Date de publication de la demande: 24.01.2018
(73) Titulaire: Pierre Fabre Dermo-Cosmétique, 92100 Boulogne-Billancourt (FR)
(72) Inventeur: LETI, Mathieu, 31450 Montgiscard (FR); MANDEAU, Anne, 31200 Toulouse (FR)
(74) Mandataire: Regimbeau
(86) Numéro de dépôt international: PCT/EP2016/056037
(87) Numéro de publication internationale: WO 2016/146837

(56) Documents cités:
- WO-A1-2009/077970
- WO-A1-2013/184884
- US-B1- 6 365 601
- WU S J ET AL: "Supercritical carbon dioxide extract exhibits enhanced antioxidant and anti-inflammatory activities of Physalis peruviana", JOURNAL OF ETHNOPHARMACOLOGY, ELSEVIER IRELAND LTD, IE, vol. 108, no. 3, 6 décembre 2006 (2006-12-06), pages 407-413, XP027939789, ISSN: 0378-8741 [extrait le 2006-12-06]
- "Product Data Sheet GLUCOPON 225 DK General characterisation Chemical description Aqueous solution of alkyl polyglucosides based on a natural fatty alcohol C8-C10 Labeling information INCI name(s) Registrations Ingredient CASR-No. EINECS/ELINCS-No. Other registrations 68515-73-1 Approved surfactant", , 31 août 2013 (2013-08-31), XP055274089, Extrait de l'Internet: URL:http://dewolfchem.com/wp-content/uploa ds/2013/08/Glucopon-225-DK-2.pdf [extrait le 2016-05-20]

## Description

Le domaine de la présente invention concerne un procédé de préparation d'un extrait de matrice végétale avec un composé amphiphile non ionique comme adjuvant d'extraction en milieu aqueux.

L'extraction solide-liquide est le procédé consistant à extraire une substance présente dans un solide, en particulier une plante, pour la faire passer dans un solvant liquide. La macération, l'infusion et la décoction sont des méthodes d'extraction solide-liquide traditionnelles.

Les solvants sont des liquides - à température d'utilisation - ayant la propriété de dissoudre, de diluer ou d'extraire d'autres substances sans les modifier chimiquement et sans eux-mêmes se modifier. Ils sont utilisés, en grande quantité, pour de nombreuses autres applications industrielles (peintures, détergents, revêtements, produits phytosanitaires...) et sont traditionnellement d'origine pétrochimique.

Cependant, la raréfaction du pétrole et la réglementation plus stricte sur les produits chimiques obligent à trouver des alternatives plus respectueuses pour l'environnement.

L'éco-extraction est basée sur la découverte et la conception de procédés d'extraction permettant, d'une part, la réduction de la consommation énergétique, mais aussi, d'autre part, le recours à des solvants alternatifs - des agro-solvants -, tout en permettant de garantir des extraits sûrs et de qualité, utilisables comme ingrédient pour la pharmacie, la cosmétique, l'agroalimentaire, la chimie fine et les bio-fuels (Green extraction of natural products (GNEP2013), (2014), Comptes Rendus Chimie, 17,179-180). Dans ce contexte, l'amélioration de procédés existants ainsi que la conception de nouveaux procédés font l'objet de nombreux travaux dans le but de réduire l'impact environnemental de l'étape d'extraction, conduisant à l'émergence et à la popularisation de technologies telles que l'extraction par ultra-sons, par micro-ondes, par CO₂ supercritique, par « flash-détente ». Parallèlement, la recherche de solvants d'extraction alternatifs, d'origine non pétrochimique, constitue une autre voie d'amélioration.

Le marché des agro-solvants est donc en plein essor, provenant du bois, des plantes de grandes cultures (amylacées ou sucrières) et espèces oléagineuses, conduisant aux dérivés terpéniques, aux alcools (éthanol, butanol, 1,3-propane diol), aux dérivés du furfural ou encore aux esters méthyliques (Formule Verte N°08 Décembre 2011, pp.28-32).

L'eau est un solvant naturel considéré comme renouvelable. Cependant, sa forte polarité ne permet pas d'extraire certaines molécules lipophiles d'intérêt.

Il est donc nécessaire de pouvoir disposer de nouveaux solvants permettant d'extraire des composés de différentes plages de polarité (gamme de polarité de plus en plus large, ou optimisation de l'extraction de composés lipophiles).

Certains composés amphiphiles non ioniques en solution aqueuse permettent, en concentration suffisante, la solubilisation de composés lipophiles.

Le nicotinamide, le diméthylisosorbide, les alkyl polyglycosides, l'urée sont des exemples de ces composés amphiphiles non ioniques. Le potentiel de ces composés comme adjuvants de solubilisation de certaines molécules lipophiles en milieu aqueux a été exploré (Sanghvi R., Evans D., Yalkowsky S. Stacking complexation by nicotinamide: a useful way of enhancing drug solubility. (2007) 336 : 35-41).

WO 2013/184884 A1 divulgue un procédé de préparation d'un extrait de plante, impliquant une extraction solide-liquide par macération à l'aide d'une solution aqueuse contenant 20% d'un alkyl-(poly)glycoside.

Mais la propriété de solubilisation n'est pas suffisante pour permettre l'extraction de solutés à partir d'une matrice végétale. En effet, dans le domaine de l'extraction végétale, le solvant d'extraction doit pénétrer dans la matrice végétale, détruire les membranes et libérer les composés dans le solvant d'imprégnation (phénomènes de diffusion, désorption, dissolution...), et permettre la diffusion du soluté de la matrice vers le film liquide entourant le solide, et le transfert vers le solvant (étape limitante). En fonction du solvant utilisé, les membranes des cellules végétales sont plus ou moins fragilisées, ce qui permet ou non la libération des composés qu'elles renferment.

La Demanderesse a mis en évidence qu'une solution aqueuse de composés amphiphiles non ioniques, utilisés en concentration suffisante, permet l'extraction de solutés de différentes plages de polarités, à partir d'une matrice végétale.

La présente invention concerne donc un procédé de préparation d'un extrait de matrice végétale impliquant une extraction solide-liquide à l'aide d'une solution aqueuse contenant au moins un composé amphiphile non ionique, de préférence agrosourcé, à une concentration au moins égale à sa concentration minimale hydrotropique.

La présente invention concerne l'obtention à l'aide d'une solution aqueuse de composés amphiphiles non ioniques soit d'un extrait total contenant des composés polaires, moyennement polaires et lipophiles, soit d'un extrait enrichi en composés lipophiles d'intérêt.

Par « composés lipophiles », on entend au sens de la présente invention, des composés avec un coefficient de partage octanol/eau, encore appelé logP ou log kow, positif.

Par « composé amphiphile non ionique », on entend selon la présente invention un composé soluble dans l'eau dans toute proportion et n'ayant pas de propriété tensioactive pour éviter la formation de micelles.

Ce procédé d'extraction est une alternative qui permet de se substituer aux solvants organiques pétrochimiques polluants, tels que l'acétate d'éthyle, l'hexane, l'acétone, etc., puisque les composés amphiphiles non ioniques préférentiels peuvent être des composés agrosourcés, i.e. provenant essentiellement de la biomasse végétale.

Le procédé selon l'invention met en oeuvre une extraction de matrice végétale à l'aide d'une solution aqueuse contenant un composé amphiphile non ionique de préférence agrosourcé, à une concentration minimale suffisante, i.e. à une concentration minimale hydrotropique.

Par « concentration minimale hydrotropique », on entend la concentration à partir de laquelle ces composés amphiphiles non ioniques commencent à former des agrégats, c'est-à-dire de nouveaux micro-environnements avec des propriétés physiques différentes de celles observées lorsque le composé est dilué, et différentes d'un comportement micellaire. Cette concentration minimale hydrotropique est spécifique à chaque composé amphiphile non ionique et est généralement de l'ordre de grandeur de la molarité. Elle peut être déterminée par plusieurs méthodes physico-chimiques, telles que la mesure de la tension de surface, la conductivité, la diffusion dynamique et statique de la lumière (Self-association of Nicotinamide in aqueous solution: Light-scattering and vapor pressure osmometry studies (1996) 85(8): 848-853) ou tout simplement en établissant une courbe de solubilisation d'un composé lipophile (teneur en soluté solubilisé en fonction de la concentration en composé amphiphile non ionique). Le rouge Soudan, colorant lipophile facilement dosable par spectrophotométrie, peut être utilisé comme référence. La valeur de cette concentration est dépendante de la nature du composé amphiphile non ionique et non du soluté. Elle correspond à la concentration minimale à partir de laquelle la courbe de solubilisation du soluté prend une allure exponentielle.

Selon une autre caractéristique de la présente invention, la solution aqueuse contenant ledit composé amphiphile non ionique, préférentiellement agrosourcé, constitue le seul solvant d'extraction utilisé.

Selon une caractéristique avantageuse de l'invention, l'extraction solide-liquide est réalisée par macération de la matrice végétale dans ladite solution aqueuse contenant au moins un composé amphiphile non ionique à concentration minimale au moins égale à la concentration minimale hydrotropique, maintenue sous agitation.

Par solution aqueuse contenant au moins un composé amphiphile non ionique à concentration au moins égale à la concentration minimale hydrotropique, on entend selon la présente invention une solution aqueuse contenant au moins un composé amphiphile non ionique à une concentration supérieure ou égale à la concentration minimale hydrotropique (CMH) précédemment rappelée. Il faut également tenir compte de la teneur en eau éventuellement présente dans la matrice végétale et ajuster en conséquence la concentration des composés amphiphiles non ioniques pour permettre leur bonne utilisation dans le procédé selon la présente invention.

Selon une autre caractéristique de l'invention, la concentration du composé amphiphile non ionique dans ladite solution aqueuse est comprise entre 1 à 10 fois, de préférence entre 1 à 6 fois, de préférence encore entre 1 à 2 fois, de manière encore plus préférentielle entre 1,4 à 1,8 fois de la concentration minimale hydrotropique. Avantageusement, dans la pratique on pourra utiliser le composé amphiphile non ionique dans une solution aqueuse à une concentration égale à 1,5 mol/L.

Selon une autre caractéristique avantageuse de la présente invention, le composé amphiphile non ionique est présent dans la solution aqueuse d'extraction à une concentration inférieure à 60 % en poids par rapport au poids de ladite solution, de préférence inférieure à 50 % en poids par rapport au poids de ladite solution, de préférence encore inférieure à 40 % en poids par rapport au poids de ladite solution de manière encore plus préférentielle inférieure à 30 % en poids par rapport au poids de ladite solution. On observera en particulier que ce seuil de concentration écarte implicitement l'utilisation de l'éthanol comme solvant d'extraction de composés lipophiles dans la mesure où, l'éthanol est en général utilisé à des teneurs très supérieures, de l'ordre de 80 %.

Selon une autre caractéristique de l'invention, ladite solution aqueuse est portée à une température pouvant aller de 20°C jusqu'au reflux pendant une durée pouvant varier de quelques minutes à plusieurs heures, selon la technique d'extraction mise en oeuvre.

Selon une autre caractéristique de l'invention, le ratio entre la matrice végétale et ladite solution aqueuse exprimées respectivement en kilogrammes et en litres, est compris entre 1 / 5 et 1 / 50.

Selon une autre caractéristique de l'invention, l'extraction solide-liquide est réalisée par tout autre système d'extraction bien connu de l'homme de l'art tels qu'une extraction sous micro-ondes, ou sous ultra-sons, ou une extraction à contre-courant, etc.).

Selon une caractéristique avantageuse de l'invention, l'extraction est suivie d'une opération de séparation solide-liquide, par filtration ou centrifugation.

Selon l'invention, ledit composé amphiphile non ionique de préférence agrosourcé, est un alkyl-(poly)glycoside de formule générale Alk-O-Zp, dans laquelle :
- Alk désigne un fragment hydrophobe aliphatique hydrocarboné, saturé ou insaturé, linéaire ou ramifié comprenant de 3 à 7 atomes de carbone, et
- Z représente un groupement glycoside hydrophile tel que le glucose, le xylose, l'arabinose, et
- 1 < p < 5

Selon un mode de réalisation particulier de l'invention, Z représente un groupement glucose.

Selon un autre mode de réalisation particulier de l'invention, Z représente un groupement xylose.

Selon encore un autre mode de réalisation particulier de l'invention, Z représente un groupement arabinose.

Selon un mode de réalisation particulier de l'invention, Alk désigne un fragment hydrophobe aliphatique hydrocarboné, saturé ou insaturé, linéaire ou ramifié comprenant 7 atomes de carbone.

Selon un autre mode de réalisation particulier de l'invention, Alk désigne un fragment hydrophobe aliphatique hydrocarboné, saturé ou insaturé, linéaire ou ramifié comprenant 6 atomes de carbone.

Selon encore un autre mode de réalisation particulier de l'invention, Alk désigne un fragment hydrophobe aliphatique hydrocarboné, saturé ou insaturé, linéaire ou ramifié comprenant 5 atomes de carbone.

Selon encore un autre mode de réalisation particulier de l'invention, Alk désigne un fragment hydrophobe aliphatique hydrocarboné, saturé ou insaturé, linéaire ou ramifié comprenant 4 atomes de carbone.

Selon une autre caractéristique de l'invention, le composé amphiphile non ionique agrosourcé est une combinaison d'un alcool gras en C7 dérivé de ricin avec du glucose de blé (sans OGM).

Selon une caractéristique avantageuse de l'invention, ledit composé est un amyl-glycoside dont le fragment hydrophobe amyl correspond à un alcool en C5 obtenu par fermentation de la betterave ou de la fécule de pomme de terre et dont le fragment glycoside provient de céréales.

Selon une caractéristique avantageuse de l'invention, ledit composé est une combinaison d'un alcool gras en C4 avec un xyloside.

Dans le cadre de la présente demande, l'abréviation APG désigne un alkyl-(poly)glucoside et l'abréviation APX désigne un alkyl-(poly)xyloside, les deux abréviations pouvant être suivies de la précision Cx, x désignant le nombre d'atomes de carbone du fragment alkyl.

Dans un autre mode de réalisation particulier de l'invention, ledit composé amphiphile non ionique de préférence agrosourcé, est un diol choisi parmi l'isopentyldiol (3-Methyl-1,3-butanediol) et/ou le méthylpropanediol ; et de préférence l'isopentyldiol.

Le composé amphiphile non ionique, utile dans le cadre de la présente invention peut avantageusement être une des matières commerciales Isopentyldiol de Kuraray, ou le Dub Diol de Stearinerie Dubois.

La présente invention concerne également l'utilisation d'une solution aqueuse contenant au moins un composé amphiphile non ionique, de préférence agrosourcé, à concentration au moins égale à une concentration minimale hydrotropique, comme solvant d'extraction solide-liquide de plantes, champignons, lichens, algues, cultures de micro-algues ou cultures de cellules végétales dédifférenciées, de préférence comme seul solvant d'extraction. L'utilisation ci-dessus concerne l'ensemble des solvants et matrices végétales mentionnés à propos du procédé de l'invention.

Par « matrice végétale », » on entend selon la présente invention tout ou partie de plante, de champignon, de lichen d'algue, de culture de micro-algues ou de culture de cellules végétales dédifférenciées.

Lesdites plante, champignon, lichen et algue sont sèches ou fraîches, congelées ou décongelées et entières (non fragmentées et non broyées), fragmentées ou broyées. Lesdites cultures de micro-algues ou de cellules végétales dédifférenciées sont entières, broyées, fraiches de préférence ou séchées, filtrées pour récupérer la biomasse, ont ou non subit un pré-traitement afin de libérer le contenu intra-cellulaire, par exemple par un procédé ultrasonique.

Par « partie de plantes », on entendra notamment les parties aériennes telles que tiges, branches, feuilles, fruits, graines et/ou fleurs ; et/ou les parties souterraines telles que les rhizomes, les racines et/ou les bulbes.

Par « partie de lichen, de champignon ou d'algue », on entendra tout organe de ces organismes tels que les thalles, les sporophores, les macromycètes, les mycéliums, et/ou les filaments.

Dans un mode de mise en oeuvre particulier de l'invention, on utilisera tout ou partie de plantes entières (non fragmentée, non broyée).

Parmi les plantes utilisables dans le cadre de la présente invention, on peut citer entre autres : les fruits de *Physalis peruviana,* les fruits d'*Embelia ribes*, les feuilles de *Myrtus communis,* les parties souterraines et les feuilles de *Piper* spp., les feuilles d'*Eucalyptus globulus,* les péricarpes de *Garcinia mangostana,* les inflorescences femelles d'*Humulus lupulus,* l'écorce de *Cinchona* sp., les parties aériennes d'*Urtica dioica,* les parties aériennes d'*Helichrysum* sp., les fruits de *Vanilla* sp., les rhizomes de *Curcuma* spp., les rhizomes de *Zingiber officinale,* les fruits et feuilles d'*Olea europaea.*

Parmi les algues utilisables dans le cadre de la présente invention, on entend en particulier les algues bleues ou Cyanobactéries ainsi que des eucaryotes parmi lesquels les Euglénophytes, Cryptophytes, Haptophytes, Gaucophytes, les algues rouges ou *Rhodophyta,* les *Stramenopiles* regroupant notamment les Diatomées et les algues brunes ou Phéophycées, et enfin les algues vertes qui comprennent entre autres les Ulvophycées.

Parmi les lichens utilisables dans le cadre de la présente invention, on peut citer entre autres : les thalles de *Cetraria islandica,* les thalles de *Usnea* spp., les thalles de *Cladonia* spp., les thalles de *Lobaria* spp.

Parmi les champignons utilisables dans le cadre de la présente invention, on peut citer entre autres *Coriolus versicolor*, *Cordyceps* spp.

Parmi les cultures de cellules végétales utilisables dans la présente invention, on peut citer entre autre : les cultures de cellules de Mimosa pudica, Tripterygium wilfordii.

A une certaine concentration, les composés amphiphiles non ioniques vont permettre, de façon inattendue, l'extraction dans l'eau de composés lipophiles. Cette concentration minimale hydrotropique est spécifique à chaque composé amphiphile non ionique et pourra être déterminée aisément par exemple par dosage spectrophotométrique de la solubilisation du rouge Soudan. Cette concentration minimale hydrotropique est généralement de l'ordre de grandeur de la molarité.

De préférence, les composés amphiphiles non ioniques sont des alkylglycosides ou alkylpolyglycosides.

Le composé amphiphile non ionique, utile dans le cadre de la présente invention, peut avantageusement être une des matières premières commerciales SEPICLEAR G7® (Société SEPPIC) et APPYCLEAN® (Société WHEATOLEO). Il peut également s'agir d'un fragment monomère de xylose présentant une fonction hydroxyle anomérique substituée par un radical alkoxy en C4.

Les conditions d'extraction (durée, concentration du composé amphiphile non ionique, pH, température, etc.), peuvent varier en fonction du couple plante / composé amphiphile non ionique de façon à optimiser le rendement et/ou la sélectivité de l'extraction. De telles adaptations spécifiques sont de la compétence de l'homme du métier faisant usage de ses connaissances générales dans le domaine de l'extraction solide-liquide.

D'une manière générale, le procédé consiste en une macération sous agitation, à des températures comprises entre 20°C et le reflux, de matrice végétale sèche ou fraîche broyée dans une solution aqueuse contenant un composé amphiphile non ionique à une concentration au moins égale à la concentration minimale hydrotropique pour permettre l'extraction des composés lipophiles.

Selon un mode de réalisation préféré, outre la solution aqueuse de composé amphiphile non ionique, aucun autre solvant n'intervient dans l'étape d'extraction proprement dite. La solution aqueuse de composé amphiphile non ionique, utilisée à une concentration spécifique, est l'unique solvant intervenant dans le procédé d'extraction.

Dans un mode de réalisation particulier de l'invention, le rapport plante (kg) / solvant (L) se situe de 1/5 à 1/50. Bien entendu, le marc peut être ré-extrait une ou plusieurs fois afin d'épuiser la plante.

L'extraction peut être réalisée dans un réacteur traditionnel ou bien assistée de tout autre système d'extraction bien connu de l'homme de l'art (micro-ondes, ultra-sons, extraction à contre-courant, etc.).

La durée d'extraction peut varier de quelques minutes à plusieurs heures, selon la technique d'extraction mise en oeuvre.

L'extraction peut se faire à partir de matrice végétale fraîche homogénéisée avec le composé amphiphile non ionique en tenant compte de la teneur en humidité de la matrice végétale.

L'extraction est suivie d'une séparation solide / liquide par centrifugation et/ou filtration.

Par « centrifugation », on entend l'action de séparer les composés d'un mélange en fonction de leur différence de densité en les soumettant à une force centrifuge, au moyen d'un décanteur centrifuge ou de tout type de centrifugeuses, afin d'obtenir une solution parfaitement limpide.

Par « filtration » on entend filtration frontale ou tangentielle, où l'on peut envisager la présence d'adjuvant de filtration (type perlite, diatomées, etc.). Cette filtration permet de retenir les derniers résidus solides, le but étant d'obtenir une solution parfaitement limpide. Elle peut être suivie d'une filtration sur membrane avec un seuil de coupure défini en fonction de la taille des molécules à considérer. Elle peut également être remplacée ou suivie par une filtration sur résine ou silice, afin d'enrichir en composé d'intérêt, par exemple en utilisant des résines d'adsorption.

Dans un mode de réalisation particulier, la solution obtenue après la séparation solide / liquide est conservée comme telle ou lyophilisée, comprenant les molécules d'intérêt, ainsi que le ou les composés amphiphiles non ioniques, lesdits composés permettant une meilleure solubilisation de l'extrait dans le produit fini.

On obtient ainsi un extrait total contenant des composés dans une large gamme de polarités (polaire, moyennement polaire, apolaire).

L'extrait ainsi obtenu peut également être dilué dans un volume d'eau additionnée ou non d'un ou plusieurs adjuvants choisis parmi des sels, acides ou bases, de façon à se situer à une concentration finale en composé amphiphile non ionique inférieure à la concentration suffisante précédemment définie. Ainsi, il est possible de récupérer les composés lipophiles par précipitation et séparation solide / liquide telle que la filtration ou la centrifugation.

On obtient ainsi un extrait enrichi en composés lipophiles. Les composés lipophiles d'intérêt peuvent être des flavonoïdes, acides phénoliques, composés terpéniques (mono-, di-, triterpénes) et stéroïdiques, des dérivés diarylheptanoïdes, des lignanes, coumarines, quinones, anthraquinones, xanthones, phloroglucinols, iridoïdes, lactones sesquiterpéniques, des alcaloïdes, des sucroesters, des lipides polaires etc.

Ils peuvent être en particulier les kavalactones, myrtucommulones, l'embeline, la quinine et ses dérivés, la vanilline et ses dérivés, l'a-mangostine, le xanthohumol, les mono et digalactosyldiacylglycérols, l'acide maslinique, l'acide ursolique, l'acide rosmarinique, le carnosol, la galangine, pinobanksine, cardamonine, les curcuminoïdes, le gingérol, shogaol.

L'extrait total ou l'extrait enrichi en composés lipophiles peuvent être dilués, concentrés, séchés ou conservés comme tel, par l'ajout de conservateur adapté et autorisé dans le produit fini souhaité (tels que les glycols, ou l'acide sorbique, l'acide benzoïque, l'acide citrique et leurs sels, etc) ou d'alcool (minimum 15°).

Pour la fourniture d'un extrait sec, les technologies de séchage sous vide, de lyophilisation ou d'atomisation peuvent être envisagées. L'extrait obtenu peut être séché avec ou sans support et / ou solubilisé dans un support liquide.

Les extraits obtenus, liquides, pâteux ou secs tels que définis plus haut peuvent être utilisés en l'état dans des compositions cosmétiques, pharmaceutiques ou alimentaires, destinées à être administrées par voie topique ou voie orale.

Les avantages sont :
- dans le cas où le filtrat est conservé comme tel ou lyophilisé, obtention d'un extrait total contenant les molécules dans une large gamme de polarités, ainsi que les composés amphiphiles non ioniques, permettant une meilleure solubilisation de l'extrait dans le produit fini.
- dans le cas où les composés lipophiles sont purifiés par précipitation, obtention d'un extrait enrichi en composés lipophiles obtenu à l'aide d'une extraction aqueuse et de substances agrosourcées, alternative aux solvants toxiques et d'origine pétrochimiques tels que l'hexane, l'acétate d'éthyle ou l'acétone.

Les exemples suivants sont donnés à titre indicatif non limitatif.

### EXEMPLES

### Exemple 1 : Extrait enrichi de Physalis peruviana

100g de fruits de physalis séchés et broyés sont agités pendant 2h à 40°C avec 700 mL d'une solution aqueuse d'heptylglucoside 1,5M (SEPICLEAR G7® commercialisé par la Société SEPPIC). Après filtration, le filtrat est acidifié à pH2 puis dilué avec 15 volumes d'eau. Après centrifugation, le culot est repris et séché. L'extrait enrichi est obtenu avec un rendement de 2.1% massique.

### Exemple 2 : Extrait enrichi de Physalis peruviana

20g de fruits de Physalis séchés et broyés sont agités pendant 2H à 40°C avec 140 mL d'une solution aqueuse d'heptylglucoside 0,75M. Après filtration, le filtrat est dilué avec 6.6 volumes d'eau. Après centrifugation, le culot est repris et séché. L'extrait enrichi est obtenu avec un rendement de 0.64% massique.

### Exemple 3 : Extrait enrichi de Physalis peruviana

20g de fruits de Physalis séchés et broyés sont agités pendant 2H à 40°C avec 140 mL d'une solution aqueuse d'heptylglucoside 3M. Après filtration, le filtrat est dilué avec 22 volumes d'eau. Après centrifugation, le culot est repris et séché. L'extrait enrichi est obtenu avec un rendement de 0.98% massique.

Résultats obtenus pour les différents extraits enrichis de Physalis :

| Extrait | Rendement | Teneur en sucroesters |
|---|---|---|
| Selon l'ex. 1 | 2.11% | 19.5% |
| Selon l'ex. 2 | 0.64% | 16.4% |
| Selon l'ex. 3 | 0.98% | 16.2% |
| Acétate d'éthyle (1 H, reflux) | 2.04% | 12.9% |
| Heptane (1 H, reflux) | 1.04% | 3.0% |

Dans les exemples 1 à 3, l'extraction des fruits de Physalis par une solution aqueuse d'heptylglucoside suivie d'une précipitation de l'extrait par dilution permet d'obtenir un extrait enrichi en sucroesters. Les sucres du fruit sont extraits mais restent en solution lors de la dilution et ne précipitent pas. La qualité des extraits obtenus est supérieure aux extraits obtenus à l'aide de solvants d'origine pétrochimiques (acétate d'éthyle, heptane).

### Exemple 4: Extrait de vanille

5 kg de gousses de vanille séchées et broyées sont extraites pendant 3h à 50°C avec 50L de solution aqueuse d'heptylglucoside 1,5M. Après filtration sous pression, le marc est rincé par 25L de la même solution. Le filtrat est concentré de façon à obtenir un extrait de vanille sous forme d'une solution sirupeuse marron-rouge.

Résultats obtenus pour l'extrait de vanille :

| Extrait | Rendement | Teneur en vanilline et dérivés |
|---|---|---|
| Selon l'Ex. 4 | 800% | 0.3% |

Cet extrait, sur support heptylglucoside, contient une proportion non négligeable de vanilline et dérivés, tout en étant pré-formulé de manière à faciliter son introduction dans la phase aqueuse d'une formule cosmétique, nutraceutique ou médicamenteuse.

### Exemple 5: Extrait enrichi de mangoustan

100 kg de péricarpes séchés et broyés de *Garcinia mangostana* sont agités pendant 2 heures à 40°C avec 1000 L d'une solution aqueuse d'heptylglucoside 1,5M. Après filtration, le filtrat est acidifié à pH = 2 puis dilué avec 11 volumes d'eau acidifiée à pH = 2. Après centrifugation, le culot est repris et séché. L'extrait enrichi en xanthones (21,3 % exprimés en α-mangostine) est obtenu avec un rendement massique de 7,3%. L'extrait obtenu est dépourvu de tanins.

En comparaison, l'extraction éthanolique à reflux de péricarpes séchés et broyés de *Garcinia mangostana* avec un rapport masse plante / volume de solvant identique donne un extrait plus pauvre en xanthones (19,5 % exprimés en α-mangostine) mais avec un rendement supérieur (27% massique). L'extrait obtenu contient des tanins.

En comparaison, l'extraction hexanique à reflux de péricarpes séchés et broyés de *Garcinia mangostana* avec un rapport masse plante / volume de solvant identique donne un extrait plus riche en xanthones (89,1 % exprimés en α-mangostine) mais avec un rendement inférieur (1,2% massique). L'extrait obtenu est dépourvu de tanins.

L'extraction avec une solution aqueuse d'heptylglucoside 1,5M permet donc une extraction sélective des xanthones en comparaison avec une extraction éthanolique. Elle permet également un rendement d'extraction plus important qu'avec une extraction hexanique.

Exemple 6 : Extrait enrichi de Piper methysticum 1 kg de parties souterraines de Piper methysticum séchées et broyées sont extraites par 700 ml d'une solution aqueuse d'amyl xyloside(APXC5) à 1,5 mol/L pendant 1 heure et 30 minutes sous agitation à 40°C. Après filtration, le filtrat est dilué par 4 volumes d'eau. Après centrifugation, le culot correspondant à l'extrait enrichi de Kava est obtenu avec un rendement de 6,2%. L'extrait contient 3,0% de kavalactones.

En comparaison, l'extraction à l'acétate d'ethyle à reflux de parties souterraines séchées et broyées de Piper methysticum avec un rapport masse plante / volume de solvant identique donne un rendement de 8,2%. L'extrait contient 5,3% de kavalactones.

En comparaison, l'extraction à l'eau à reflux de parties souterraines séchées et broyées de Piper methysticum avec un rapport masse plante / volume de solvant identique donne un rendement de 23,1%. L'extrait contient 0.25% de kavalactones.

Les trois extraits ont une composition en kavalactones différente :
- L'extrait obtenu par extraction par la solution aqueuse d'amyl glycosides à 1,5 mol/L est plus riche en kavalactones peu polaires (yangonine, déméthoxyyangonine, flavokawaines A,B et C) que l'extrait acétate d'éthyle (44,8 % contre 35,3% des kavalactones totales).
- A l'inverse, l'extrait obtenu par extraction par la solution aqueuse d'amyl glycosides à 1,5 mol/L est plus pauvre en kavalactones les plus polaires (méthysticine, dihydroxyméthysticine, kawaine et marindinine) que l'extrait acétate d'éthyle (55,2% contre 64,7% des kavalactones totales).
- L'extrait obtenu par extraction aqueuse contient 1,66% de kavalactones et ne contient pas de kavalactones peu polaires (yangonine, déméthoxyyangonine, flavokawaines A, B et C).

L'extraction avec une solution aqueuse d'amyl glycosides à 1,5 mol/L permet donc une extraction sélective des kavalactones les moins polaires pour un rendement total comparable à l'extraction par acétate d'éthyle.

### Exemple 7 : gélule

| | |
|---|---|
| Extrait de mangoustan selon l'exemple 5 | 200 mg |
| Amidon | 45 mg |
| Stéarate de magnésium | 2 mg |

### Exemple 8 : crème

| | |
|---|---|
| Extrait de vanille selon l'exemple 4 | 0,5-3 % |
| Tribehenin PEG- 20 esters | 2-7 % |
| Isodecyl neopentanoate | 2-9 % |
| Glycérine | 0.5-10% |
| Glycol palmitate | 1-6 % |
| Cetyl alcohol | 0.5 - 3% |
| Disodium EDTA | 0.05-0.25 % |
| Conservateurs | 0.5-3 % |
| Parfum | 0.2-0.5 % |
| Xanthan gum | 0.1-0.4 % |
| Eau | qs |

### Exemple 9 : Exemples de courbes de solubilisation

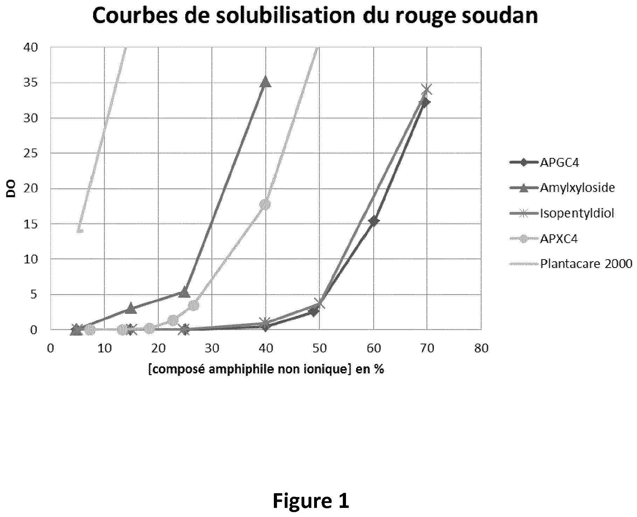

Les courbes de solubilisation du rouge Soudan dans des solutions aqueuses de différents composés amphiphiles non ioniques à des concentrations différentes ont été réalisées. Après saturation des solutions au rouge Soudan et filtration, la teneur de rouge Soudan solubilisé dans chaque solution est mesurée après dilution, par lecture au spectrophotomètre UV à 476 nm. Les courbes de solubilisation apparaissent en figure 1 annexée. En ordonnée sont reprises les valeurs de DO multipliées par la dilution, ces valeurs étant proportionnelles à la concentration (selon la loi de Beer-Lambert).

Ces courbes permettent de déterminer la concentration minimale hydrotropique des composés amphiphiles non ioniques selon l'invention.

Le Plantacare (décylglucoside) ne convient pas, ayant un comportement clairement tensio-actif (forte solubilisation à faible concentration, le composé formant des micelles). Il serait impossible de récupérer les composés d'intérêt par dilution. De plus, la formation de mousse apportée par le tensio-actif gêne considérablement la filtration.

L'allure des courbes obtenues avec l'a-mangostine selon un procédé similaire (dosage de l'a-mangostine solubilisée par HPLC) sont comparables à celles obtenues avec le rouge soudan, démontrant que cette valeur dépend du composé amphiphile et non du soluté.

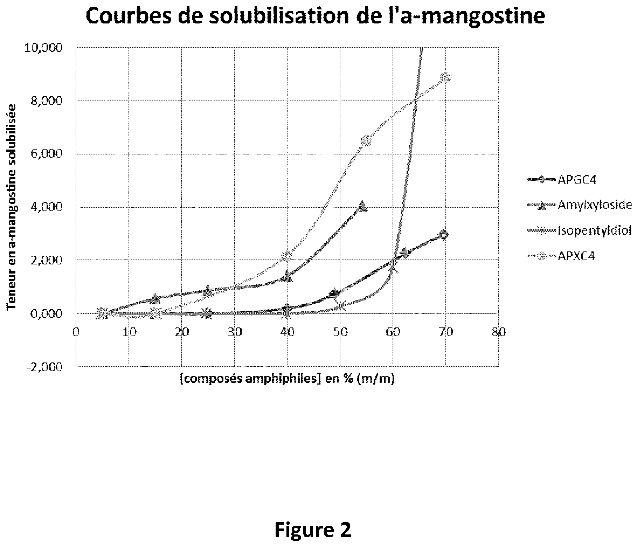

Les courbes de solubilisation de l'a-mangostine dans des solutions aqueuses de composés amphiphiles non ioniques à différentes concentrations apparaissent à la figure 2 annexée.

On peut ainsi déduire de ces courbes que l'APXC4 possède une CMH de 15-20%, et l'amylxyloside de 5-10%.
La CMH de l'isopentyldiol est comprise entre 40 et 45%.

Ces valeurs sont nécessaires pour la mise en oeuvre du procédé d'extraction, comme par exemple l'extraction d'a-mangostine à partir des péricarpes de mangoustan :

| **Composé** | **Concentration** | **g d'a-mangostine extraite % g de plante** |
|---|---|---|
| **APX C4** | 5% | 0,36 |
| **APX C4** | 25% | 7,87 |
| **Isopentyldiol** | 59% | 6,59 |
| **Amylxyloside** | 40% | 7,30 |
| **AcOEt** | 100% | 7,49 |

A 25% d'APXC4, la teneur en principe actif extraite des péricarpes de mangoustan est équivalente à celle obtenue par extraction à reflux à l'acétate d'éthyle. Ce qui n'est pas le cas à 5% (concentration inférieure à la CMH).

Ce principe actif peut ensuite être récupéré par dilution lorsque la concentration finale en composé amphiphile est inférieure à la CMH, comme on peut le voir dans le tableau ci-dessous :

| Composé | Concentration d'extraction | Concentration , après dilution | % d'a-mangostine récupérée par précipitation / a-mangostine extraite |
|---|---|---|---|
| APX C4 | 25% | 9% | 86% |
| Isopentyldiol | 59% | 24% | 66% |
| Amylxyloside | 40% | 15% | 12% |
| | | 7% | 100% |

⇒ En diluant la solution d'amylxyloside à 15%, peu d'a-mangostine précipite, la concentration restant supérieure à la CMH. A 7% d'amylxyloside, 100% de l'a-mangostine extraite précipite.

### Exemple 10 :

### - Extraction de grignons d'olive frais

Peser 10 g de pulpe d'olive broyée après pressage pour récupérer l'huile (contenant 76% de teneur en eau), y rajouter l'équivalent de 12 g d'APXC4 en matière sèche et de l'eau de façon à se situer à une concentration finale en APXC4 de 50% (en tenant compte de la teneur en eau de la plante). Chauffer 3H à 50°C et filtrer afin de récupérer un filtrat limpide marron avec un rendement de 87.5%.

Parallèlement, lyophiliser cette même pulpe d'olive (rendement 24.5%) et l'extraire à l'acétate d'éthyle pendant 1H à reflux. Après évaporation du solvant, on obtient une huile trouble verte avec un rendement de 23.4% / matière sèche et 5.7% / matière fraîche.

Evaluation des extraits obtenus par CCM dans les conditions suivantes:
- Phase stationnaire : Plaque CCM recouverte de gel de silice 60
- Phase mobile : Acétate d'éthyle / Cyclohexane (1:1)
- Révélateur : Vanilline sulfurique + chauffage à 120°C

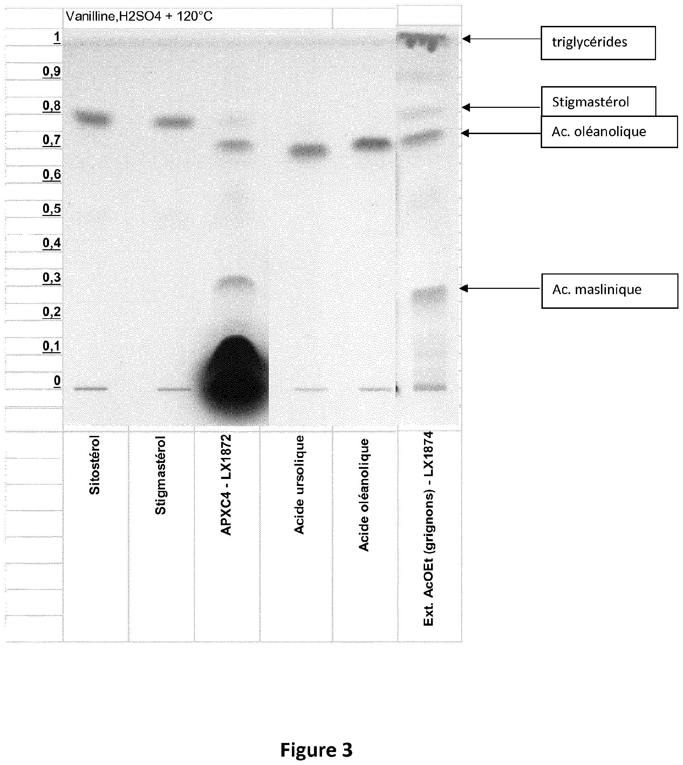

La comparaison des profils chromatographiques CCM apparaissant en figure 3 annexée montre que l'extrait (LX 1872) avec l'APXC4 contient bien les triterpènes (acide oléanolique et acide maslinique), alors que l'extrait AcOEt (LX 1874) contient des triglycérides en plus des triterpènes. Les molécules d'intérêt sont donc présentes sans avoir à sécher la matière et à l'extraire avec un solvant toxique et volatile. De plus, on n'extrait ni la chlorophylle, ni les lipides neutres.

## Revendications

1. Procédé de préparation d'un extrait de matrice végétale, en particulier de plante impliquant une extraction solide-liquide à l'aide d'une solution aqueuse contenant au moins un composé amphiphile non ionique, de préférence agrosourcé, à une concentration au moins égale à sa concentration minimale hydrotropique, **caractérisé en ce que** ledit composé amphiphile non ionique est un alkyl-(poly)glycosides de formule générale Alk-O-Zp, dans laquelle :
• Alk désigne un fragment hydrophobe aliphatique hydrocarboné, saturé ou insaturé, linéaire ou ramifié comprenant de 3 à 7 atomes de carbone, et
• Z représente un groupement glycoside hydrophile tel que le glucose, le xylose et l'arabinose, et
• 1 < p < 5

2. Procédé selon la revendication 1, **caractérisé en ce que** la solution aqueuse contenant ledit composé amphiphile non ionique constitue le seul solvant d'extraction utilisé.

3. Procédé selon l'une des revendications 1 à 2, **caractérisé en ce que** l'extraction solide-liquide est réalisée par macération de la plante dans ladite solution aqueuse contenant au moins un composé amphiphile non ionique, maintenue sous agitation.

4. Procédé selon l'une des revendications 1 à 2, **caractérisé en ce que** l'extraction solide-liquide est réalisée sous micro-ondes, sous ultra-sons ou bien à contre-courant.

5. Procédé selon la revendication 4, **caractérisé en ce que** la concentration hydrotropique minimale du composé amphiphile non ionique dans ladite solution aqueuse est comprise entre 1 à 10 fois, de préférence entre 1 à 6 fois, de préférence encore entre 1 à 2 fois, de manière plus préférentielle entre 1,4 à 1,8 fois de la concentration minimale hydrotropique.

6. Procédé selon l'une des revendications 1 à 5, **caractérisé en ce que** le composé amphiphile non ionique est présent dans ladite solution aqueuse à une concentration inférieure à 60 % en poids par rapport au poids de ladite solution, de préférence inférieure à 50 % en poids par rapport au poids de ladite solution, de préférence encore inférieure à 40 % en poids par rapport au poids de ladite solution, de manière encore plus préférentielle inférieure à 30 % en poids par rapport au poids de ladite solution aqueuse.

7. Procédé selon l'une des revendications 1 à 6, **caractérisé en ce que** ladite solution aqueuse est portée à une température allant de 20°C jusqu'au reflux pendant une durée pouvant varier de quelques minutes à plusieurs heures, selon la technique d'extraction mise en œuvre.

8. Procédé selon l'une des revendications 1 à 7, **caractérisé en ce que** le ratio entre la plante et ladite solution aqueuse exprimées respectivement en kilogrammes et en litres, est compris entre 1 / 5 et 1 / 50.

9. Procédé selon l'une des revendications 1 à 8, **caractérisé en ce que** l'extraction est suivie d'une opération de séparation solide-liquide, par filtration ou centrifugation.

10. Procédé selon l'une des revendications 1 à 9, **caractérisé en ce que** le composé amphiphile non ionique est une combinaison d'un alcool gras en C₇ dérivé de ricin avec du glucose de blé (sans OGM).

11. Procédé selon l'une des revendications 1 à 9, **caractérisé en ce que** ledit composé est un amyl-glycoside dont le fragment hydrophobe amyl correspond à un alcool en C₅ obtenu par fermentation de la betterave ou de la fécule de pomme de terre et dont le fragment glycoside provient de céréales.

12. Procédé selon l'une des revendications précédentes, **caractérisé en ce que** la plante utilisée est choisie parmi les fruits de *Physalis peruviana,* les fruits d'*Embelia ribes,* les feuilles de Myrtus *communis,* les parties souterraines et les feuilles de *Piper* spp., les feuilles d'*Eucalyptus globulus,* les péricarpes de *Garcinia mangostana,* les inflorescences femelles d'*Humulus lupulus,* l'écorce de *Cinchona* sp., les parties aériennes d'*Urtica dioica,* les parties aériennes d'*Helichrysum* sp., les fruits de *Vanilla* sp., les rhizomes de *Curcuma* spp., les rhizomes de *Zingiber officinale* et les fruits et feuilles *d'Olea europaea.*

13. Procédé selon l'une des revendications 9 à 12, **caractérisé en ce que** la solution obtenue après séparation solide /liquide est conservée comme telle ou lyophilisée,, comprenant les molécules d'intérêt, ainsi que les composés amphiphiles non ioniques, lesdits composés amphiphiles non ioniques permettant une meilleure solubilisation de l'extrait dans le produit fini.

14. Procédé selon l'une des revendications précédentes, **caractérisé en ce que** dans l'extrait obtenu, les composés lipophiles sont purifiés par précipitation.

## Patentansprüche

1. Verfahren zur Herstellung eines Pflanzenmatrixextrakts, insbesondere aus einer Pflanze, das eine Fest-Flüssig-Extraktion mit Hilfe einer wässrigen Lösung impliziert, die zumindest eine bevorzugt aus dem Pflanzenbau kommende nichtionische amphiphile Verbindung in einer Konzentration enthält, die zumindest gleich ihrer hydrotropen Mindestkonzentration ist, **dadurch gekennzeichnet, dass** die genannte nichtionische amphiphile Verbindung ein Alkyl(poly)glykosid mit der allgemeinen Formel Alk-O-Zp ist, in der:
• Alk ein hydrophobes, aliphatisches kohlenwasserstoffhaltiges, gesättigtes oder ungesättigtes, lineares oder verzweigtes Fragment bezeichnet, das 3 bis 7 Kohlenstoffatome umfasst, und
• Z für eine hydrophile Glykosidgruppe wie Glukose, Xylose und Arabinose steht, und
• 1<p<5.

2. Verfahren nach Anspruch 1, **dadurch gekennzeichnet, dass** die wässrige Lösung, die diese nichtionische amphiphile Verbindung enthält, das einzige verwendete Extraktionslösungsmittel ist.

3. Verfahren nach einem der Ansprüche 1 bis 2, **dadurch gekennzeichnet, dass** die Fest-Flüssig-Extraktion durch Mazeration der Pflanze in der wässrigen Lösung durchgeführt wird, die zumindest eine nichtionische amphiphile Verbindung enthält, die konstant gerührt wird.

4. Verfahren nach einem der Ansprüche 1 bis 2, **dadurch gekennzeichnet, dass** die Fest-Flüssig-Extraktion unter Mikrowellen, Ultraschall oder mit Gegenstrom durchgeführt wird.

5. Verfahren nach Anspruch 4, **dadurch gekennzeichnet, dass** die minimale hydrotropische Konzentration der nichtionischen amphiphilen Verbindung in der wässrigen Lösung zwischen dem 1- bis 10-Fachen, vorzugsweise zwischen dem 1- bis 6-Fachen, weiter vorzugsweise zwischen dem 1- bis 2-Fachen, noch weiter vorzugsweise zwischen dem 1,4- bis 1,8-Fachen der minimalen hydrotropischen Konzentration liegt.

6. Verfahren nach einem der Ansprüche 1 bis 5, **dadurch gekennzeichnet, dass** die nichtionische amphiphile Verbindung in der wässrigen Lösung in einer Konzentration von weniger als 60 Gew.-% im Verhältnis zum Gewicht der Lösung, vorzugsweise von weniger als 50 Gew.-% im Verhältnis zum Gewicht der Lösung, weiter vorzugsweise von weniger als 40 Gew.-% im Verhältnis zum Gewicht der Lösung, noch weiter vorzugsweise von weniger als 30 Gew.-% im Verhältnis zum Gewicht der wässrigen Lösung vorhanden ist.

7. Verfahren nach einem der Ansprüche 1 bis 6, **dadurch gekennzeichnet, dass** die wässrige Lösung während einer Zeitdauer, die je nach der angewandten Extraktionstechnik von einigen Minuten bis zu mehreren Stunden variieren kann, auf eine Temperatur zwischen 20°C und dem Rückfluss erhitzt wird.

8. Verfahren nach einem der Ansprüche 1 bis 7, **dadurch gekennzeichnet, dass** das Verhältnis zwischen der Pflanze und der wässrigen Lösung in Kilogramm beziehungsweise Liter ausgedrückt zwischen 1:5 und 1:50 liegt.

9. Verfahren nach einem der Ansprüche 1 bis 8, **dadurch gekennzeichnet, dass** auf die Extraktion eine Fest-Flüssig-Trennung durch Filtern oder Zentrifugieren folgt.

10. Verfahren nach einem der Ansprüche 1 bis 9, **dadurch gekennzeichnet, dass** die nichtionische amphiphile Verbindung eine Kombination eines von Rizinus abgeleiteten C₇-Fettalkohols mit Weizenglukose (GVOfrei) ist.

11. Verfahren nach einem der Ansprüche 1 bis 9, **dadurch gekennzeichnet, dass** die Verbindung ein Amylglykosid ist, dessen hydrophobes Amylfragment einem C₅-Alkohol entspricht, der durch Fermentation von Rüben- oder Kartoffelstärke gewonnen wird, und dessen Glykosidfragment aus Getreide stammt

12. Verfahren nach einem der vorstehenden Ansprüche, **dadurch gekennzeichnet, dass** die verwendete Pflanze aus den Früchten von *Physalis peruviana* (Kapstachelbeeren), den Früchten von *Embelia ribes,* den Blättern von *Myrtus communis* (Myrtus), den unterirdischen Teilen und den Blättern von *Piper* spp. (Pfeffer), den Blättern von *Eucalyptus globulus,* den Perikarpen von *Garcinia mangostana* (Mangostanbaum), den Fruchtzapfen weiblicher von *Humulus lupulus* (Hopfenpflanzen), der Rinde von *Cinchona* sp., den oberirdischen Teilen von *Urtica dioica* (Brennesse), den oberirdischen Teilen von *Helichrysum* sp., den Früchten von *Vanilla* (Vanille) sp., den Rhizomen von *Curcuma* spp. (Curcuma SPP), den Rhizomen von *Zingiber officinale* und den Früchten und Blättern von *Olea europaea* (Olivenbaum) ausgewählt ist.

13. Verfahren nach einem der Ansprüche 9 bis 12, **dadurch gekennzeichnet, dass** die nach der Fest-Flüssig-Trennung erzielte Lösung als solche oder lyophilisiert aufbewahrt wird, die die interessierenden Moleküle sowie die nichtionischen amphiphilen Verbindungen enthält, wobei diese nichtionischen amphiphilen Verbindungen eine bessere Solubilisierung des Extrakts im Endprodukt ermöglichen.

14. Verfahren nach einem der vorstehenden Ansprüche, **dadurch gekennzeichnet, dass** im erzielten Extrakt die lipophilen Verbindungen durch Ausfällung gereinigt werden.

## Claims

1. A method for producing a vegetable matrix extract, in particular a plant extract, involving solid-liquid extraction with the aid of an aqueous solution containing at least one non-ionic amphiphilic compound, preferably agro-sourced, at a concentration at least equal to its minimum hydrotrope concentration, **characterized in that** said non-ionic amphiphilic compound is an alkyl-(poly)glycoside of general formula Alk-O-Zp, wherein:
• Alk denotes a saturated or unsaturated, linear or branched hydrophobic aliphatic hydrocarbon fragment having 3 to 7 carbon atoms, and
• Z is a hydrophilic glycoside group such as glucose, xylose and arabinose, and
• 1<p<5

2. The method as claimed in claim 1, **characterized in that** the aqueous solution containing said non-ionic amphiphilic compound is the only extraction solvent used.

3. The method as claimed in one of claims 1 to 2, **characterized in that** the solid-liquid extraction is performed by maceration of the plant in said aqueous solution containing at least one non-ionic amphiphilic compound, maintained under stirring.

4. The method as claimed in one of claims 1 to 2, **characterized in that** the solid-liquid extraction is performed under microwaves, under ultrasound or in a countercurrent process.

5. The method as claimed in claim 4, **characterized in that** the minimum hydrotrope concentration of the non-ionic amphiphilic compound in said aqueous solution is between 1 to 10 times, preferably between 1 to 6 times, more preferably between 1 to 2 times, even more preferably between 1.4 and 1.8 times the minimum hydrotrope concentration.

6. The method as claimed in one of claims 1 to 5, **characterized in that** the non-ionic amphiphilic compound is present in said aqueous solution at a concentration of less than 60% by weight relative to the weight of said solution, preferably less than 50% by weight relative to the weight of said solution, more preferably less than 40% by weight relative to the weight of said solution, even more preferably less than 30% by weight relative to the weight of said aqueous solution.

7. The method as claimed in one of claims 1 to 6, **characterized in that** said aqueous solution is heated to a temperature ranging from 20°C to reflux for a period of time varying from several minutes to several hours, depending on the extraction technique used.

8. The method as claimed in one of claims 1 to 7, **characterized in that** the ratio between the plant and said aqueous solution, expressed respectively in kilograms and in liters, is between 1/5 and 1/50.

9. The method as claimed in one of claims 1 to 8, **characterized in that** the extraction is followed by solid-liquid separation by filtration or centrifugation.

10. The method as claimed in one of claims 1 to 9, **characterized in that** the non-ionic amphiphilic compound is a combination of a C₇ fatty alcohol derived from *Ricinus* and with wheat glucose (non-GMO).

11. The method as claimed in one of claims 1 to 9, **characterized in that** said compound is an amyl glycoside whose hydrophobic amyl fragment corresponds to a C₅ alcohol obtained by fermentation of beet or of potato flour and whose glycoside fragment is derived from cereals.

12. The method as claimed in one of the preceding claims, **characterized in that** the plant used is selected from the fruits of *Physalis peruviana,* the fruits of *Embelia ribes,* the leaves of *Myrtus communis,* the underground parts and the leaves of *Piper* spp., leaves of *Eucalyptus globulus,* the pericarps of *Garcinia mangostana,* the female inflorescences of *Humulus lupulus,* the bark of *Cinchona* sp., the aerial parts of *Urtica dioica*, the aerial parts of *Helichrysum* sp., the fruits of *Vanilla* sp., the rhizomes of *Curcuma* spp., the rhizomes of *Zingiber officinale* and the fruits and the leaves of *Olea europaea.*

13. The method as claimed in one of claims 9 to 12, **characterized in that** the solution obtained after solid-liquid separation is preserved as such or is lyophilized, including the molecules of interest, as well as the non-ionic amphiphilic compounds, said non-ionic amphiphilic compounds allowing better solubilization of the extract in the final product.

14. The method as claimed in one of the preceding claims, **characterized in that** in the extract obtained, the lipophilic compounds are purified by precipitation.
